# FASCICULE DE BREVET EUROPEEN

(11) **EP 4 433 290 B1**
(45) Date de publication et mention de la délivrance du brevet: **31.12.2025**
(21) Numéro de dépôt: 22817204.5
(22) Date de dépôt: 10.11.2022
(51) Int. Cl.: B29D 30/00, G01N 21/552, G01N 21/84

(54) **PROCÉDÉ ET SYSTÈME DE CONTRÔLE DE FABRICATION DE PRODUITS CAOUTCHOUTEUX EN RÉPONSE AUX PROPRIÉTÉS PHYSICO-CHIMIQUES D'UN MÉLANGE CAOUTCHOUTEUX**
VERFAHREN UND SYSTEM ZUR STEUERUNG DER HERSTELLUNG VON KAUTSCHUKPRODUKTEN ALS REAKTION AUF DIE PHYSIKALISCH-CHEMISCHEN EIGENSCHAFTEN EINER KAUTSCHUKVERBINDUNG
METHOD AND SYSTEM FOR CONTROLLING THE MANUFACTURE OF RUBBER PRODUCTS IN RESPONSE TO THE PHYSICOCHEMICAL PROPERTIES OF A RUBBER COMPOUND

(30) Priorité: 16.11.2021 FR 2112099
(43) Date de publication de la demande: 25.09.2024
(73) Titulaire: COMPAGNIE GENERALE DES ETABLISSEMENTS MICHELIN, 63000 Clermont-Ferrand (FR)
(72) Inventeur: MONTOY, Aurelien, 63040 CLERMONT-FERRAND CEDEX 09 (FR)
(74) Mandataire: M.F.P. Michelin
(86) Numéro de dépôt international: PCT/EP2022/081479
(87) Numéro de publication internationale: WO 2023/088778

(56) Documents cités:
- WO-A1-2021/011397
- FR-A1- 3 091 346

## Description

### Domaine Technique

L'invention concerne l'utilisation des mesures sensiblement « olfactives » dans les installations où des produits caoutchouteux (y compris des pneumatiques) sont fabriqués à partir des mélanges caoutchouteux. Plus particulièrement, l'invention concerne des systèmes et des procédés pour déterminer des corrélations entre un ou des phénomènes physiques liés à un caoutchouc (par exemple, le collant) et les évolutions des composés organo-volatiles provenant des mélanges caoutchouteux.

### Contexte

Le domaine de fabrication des produits caoutchouteux (y compris des pneumatiques) à partir de mélanges caoutchouteux implique des procédés comprenant multiples étapes de transformation des matières premières, y compris, sans limitation, des étapes de broyage et de lavage, des étapes de convection chaude pour le séchage des miettes de caoutchouc humide, l'emballage du caoutchouc séché, et le stockage de caoutchouc fabriqué. Les émissions de des composés organiques volatils (ou « COV ») des caoutchoucs différents exposent un nombre d'odorants en termes de concentrations ainsi que de type d'odorant. Pendant ces procédés, la surveillance des COV présente une opportunité importante pour évaluer les changements dans la composition des COV émis pendant le traitement du caoutchouc.

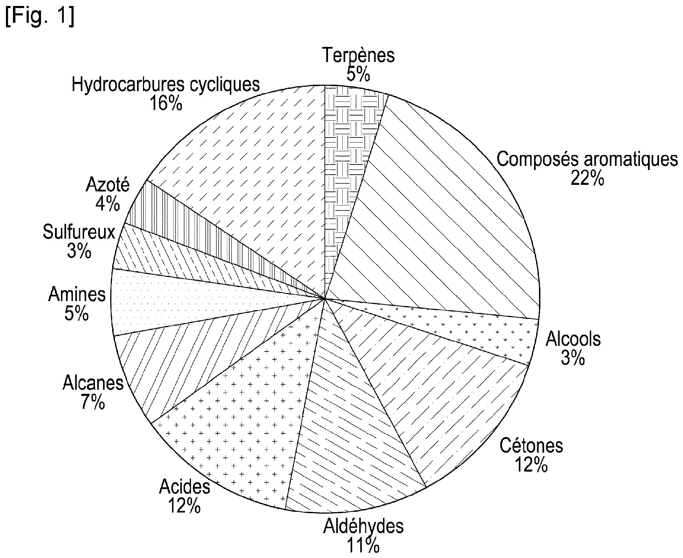

Dans une étude qui compare les COV émis directement à partir des matières premières du caoutchouc ou du caoutchouc non transformé, les composés identifiés ont été classés dans différents groupes chimiques (par exemple, acides, alcools, composés aromatiques, aldéhydes, alcanes, éthers, esters, hydrocarbures cycliques, cétones, sulfureux, azotés, terpènes, et autres)(voir « Quantification of VOCs and the Development of Odour Wheels for Rubber Processing », Nor H. Kamarulzaman, Nhat Le-Minh, Ruth M. Fisher, Richard M. Stuetz, Science of the Total Environment 657 (2019))(« la référence Kamarulzaman »)). Dans cette étude, certains des odorants ont été soumis à une analyse comparative des COV afin d'identifier les tendances générales du comportement des émissions de caoutchouc. D'après cette étude, la variation des odeurs identifiées peut être associée à des propriétés spécifiques du caoutchouc, en particulier les niveaux de protéines et le taux d'humidité. A titre d'exemple, la Figure 1, correspondant à la Figure 8 de la référence Kamarulzaman, représente l'ensemble des groupes chimiques obtenus à partir d'un caoutchouc chauffé (voir aussi le Tableau 3 de la référence Kamarulzaman). Le groupe dominant de COV libérés par l'échantillon chauffé étaient les composés aromatiques, suivis des cétones, des aldéhydes, des acides et des composés de type cyclique. Les groupes sulfureux et azotés présentaient un pourcentage faible et similaire d'odorants, cependant ils étaient susceptibles de contribuer au profil olfactif.

Il existe dans le commerce des outils visant à reconnaître la présence d'un composé cible (par exemple, un analyte chimique ou biologique) dans un échantillon gazeux. Parmi ces outils, des nez électroniques emploient souvent un ou des capteurs qui mesurent la concentration d'une substance par résonance plasmonique de surface (connu dans l'art comme « SPR » ou « Surface Plasmon Resonance » en anglais)(voir « Highly-Selective Optoelectronic Nose Based on Surface Plasmon Resonance Imaging for Sensing Volatile Organic Compounds », Sophie Brenet, Aurelian John-Herpin, François-Xavier Gallat, Benjamin Musnier, Arnaud Buhot, Cyril Herrier, Tristan Rousselle, Thierry Livache, and Yanxia Hou, Analytical Chemistry, 90, 9879-9887 (2018). La technique SPR peut mesurer l'interaction entre les molécules en utilisant des principes optiques sans substance de marquage séparée telle qu'un matériau fluorescent. Les plasmons de surface sont des vibrations quantifiées d'électrons libres se propageant le long de la surface d'un conducteur, tel qu'une surface métallique. Ces plasmons de surface traversent un milieu diélectrique tel qu'un prisme et pénètrent dans un film métallique à un angle supérieur à l'angle critique du milieu diélectrique. Ils sont excités par la lumière incidente et provoquent une résonance à un certain angle. L'angle d'incidence auquel cette résonance se produit (appelé « l'angle de résonance ») est sensible aux variations de l'indice de réfraction d'un matériau proche du film métallique.

La SPR comprend une technique connue permettant de détecter un changement local d'indice optique (étant un indice de réfraction) qui caractérise l'interaction du composé cible avec chaque capteur du nez électronique. Les capteurs SPR peuvent analyser quantitativement les échantillons à partir du changement de l'indice de réfraction du matériau proche du film métallique (c.-à-d., un échantillon utilisant les propriétés ci-dessus). Pour l'analyse de COV, l'efficacité de la SPR contribue aux bonnes performances des nez électroniques, telles que leur sensibilité, leur sélectivité, leur répétabilité de performance et leur stabilité. Il existe aussi des solutions d'imagerie par SPR pour la détection des COV en phase gazeuse qui présentent une bonne répétabilité et une bonne stabilité (voir les solutions divulguées par les publications FR3063543, WO2021/009440, WO2021/053284 et WO2021/053285 et offertes dans le commerce par Aryballe Technologies).

Le document de brevet FR 3 091 346 A1 décrit un procédé de caractérisation de composés cibles, présents dans un échantillon fluide par un nez électronique utilisant une technologie d'imagerie par résonance plasmonique de surface.

En outre, l'amélioration récente des techniques d'apprentissage machine et de l'analyse des données, combinée à des plateformes de calcul et de stockage de données, a ouvert des voies pour le développement de nouvelles approches pour lier les profils olfactifs et les solutions utilisant l'effet SPR. Dans le domaine de l'intelligence artificielle (ou « IA »), les techniques d'apprentissage automatiques sont connues, et leur fondement est d'être « entraîné » sur un grand nombre de situations. Grâce à l'ajustement des coefficients de pondération dans une phase d'apprentissage, les performances de l'apprentissage automatiques peuvent prévoir le résultat d'une situation nouvelle qui serait présentée. Il est entendu que plusieurs méthodes d'apprentissage distinctes sont possibles, y compris l'apprentissage supervisé (dans lequel l'algorithme s'entraîne sur un ensemble de données étiquetées et se modifie jusqu'à être capable d'obtenir le résultat souhaité), l'apprentissage non-supervisé ou semi-supervisé (dans lequel les données ne sont pas étiquetées pour que le réseau puisse s'adapter pour augmenter la précision de l'algorithme), l'apprentissage renforcé (dans lequel l'algorithme est renforcé pour les résultats positifs et sanctionné pour les résultats négatifs) et l'apprentissage progressif (l'algorithme demande au fur et à mesure des exemples et labels pour raffiner sa prédiction)(voir https://www.lebigdata.fr/reseau-de-neurones-artificiels-definition).

En cherchant à évaluer des paramètres d'un produit caoutchouteux, certains ne sont pas accessibles par des moyens de mesure. Par exemple, le vieillissement est actuellement géré par des dates de péremption empiriques. Concernant le collant, les ateliers utilisent principalement des Tackmetres à billes permettant d'estimer le collant d'un produit, nécessitant la prise d'échantillon. Dans les deux cas, la mesure est soumise à l'effet « opérateur » en plus des autres erreurs de mesures.

Ainsi, l'établissement d'un lien entre les odeurs et les substances odorantes peut en profiter l'impact des odeurs dans la fabrication des produits caoutchouteux et de développer des pratiques efficaces de production et de gestion sur un site de production de produits caoutchouteux. L'invention divulguée associe donc les données SPR, obtenus par des nez électroniques, et les installations de production des produits caoutchouteux pour en profiter les propriétés physico-chimiques connues dans le domaine de production de produits caoutchouteux (par exemple, des odeurs caractéristiques influençant le collant des mélanges sur les procédés industriels) (comme utilisés ici, les termes « mélange » et « mélange caoutchouteux » sont interchangeables). En utilisant un système à base intelligence artificielle, les données obtenues permettent d'établir un lien non évident entre l'aspect physico-chimique SPR et de l'aspect olfactif du ou des produits caoutchouteux en cours de production.

### Résumé de l'invention

L'invention concerne un procédé de contrôle de fabrication de produits caoutchouteux réalisé par un système de fabrication de produits caoutchouteux comprenant une installation de production ayant au moins un moyen de mélangeage qui met en œuvre des étapes successives de mélangeage d'un mélange caoutchouteux, caractérisé en ce que le procédé comprend les étapes suivantes :
- une étape de démarrage d'un cycle de mélangeage réalisé à l'installation de production;
- une étape de capture d'air ambient autour de l'installation de production pour obtenir au moins un échantillon gazeux pendant le cycle de mélangeage, cette étape étant réalisée par un dispositif de détection d'odeur du système qui reconnaît la présence des composés organiques volatiles odorants présents dans l'air ambiant reçu dans le dispositif en mesurant sa concentration par résonance plasmonique de surface ;
- une étape de détection de profils olfactifs présents dans l'air ambient et capturés par le dispositif ;
- une étape d'identification d'un profil olfactif pour identifier un mélange sortant du moyen de mélangeage et son état de production, cette étape étant réalisée à partir d'au moins un échantillon de l'air ambient capturée par le dispositif ;
- une étape d'analyse des échantillons capturés par le dispositif à partir d'une ou des empreintes SPR fournies par le dispositif et les données extraites représentées dedans;
- une étape de construction d'au moins un modèle de profils olfactifs à partir des propriétés physico-chimiques du mélange caoutchouteux identifié, cette étape comprenant une etape d'introduction à un réseau neuronal des propriétés physico-chimiques du mélange caoutchouteux identifié, cette étape étant réalisée par le système ; et
- une étape d'entrainement du modèle de profils olfactifs à partir des profils olfactifs du mélange caoutchouteux identifié;
de sorte que le modèle de profils olfactifs sortant sera l'identification des propriétés physico-chimiques du mélange caoutchouteux en cours de production à l'installation de production.

Dans des modes de réalisation du procédé de l'invention, l'étape de construction du modèle de profils olfactifs comprend une étape de création d'une référence des profils olfactifs cherchés dans l'air ambient d'être capturé par le dispositif.

Dans des modes de réalisation du procédé de l'invention, l'étape d'introduction de l'étape de construction du modèle de profils olfactifs comprend une étape de création d'une base d'apprentissage des propriétés physico-chimiques qui est introduite dans le modèle de profils olfactifs.

Dans des modes de réalisation du procédé de l'invention, le procédé comprend en outre une étape de comparaison pendant laquelle l'identification des propriétés physico-chimiques du mélange sortant du moyen de mélangeage, ressorti du modèle de profils olfactifs, est comparé au profil olfactif du mélange identifié, de sorte que le système puisse ajuster l'installation de production où les propriétés souhaitées du mélange ne sont pas atteintes.

Dans des modes de réalisation du procédé de l'invention, le procédé comprend en outre une étape de commande d'ajustement de l'installation de production pour prévoir l'atteint des propriétés souhaitées du mélange sortant du moyen de mélangeage. Dans tels modes de réalisation du procédé de l'invention, l'étape de commande d'ajustement de l'installation de production comprend une étape de détermination d'un niveau de collant du mélange à partir d'une empreinte SPR fournie par le dispositif.

Dans des modes de réalisation du procédé de l'invention, pendant l'étape d'entrainement du modèle de profils olfactifs, le système emploie une méthode d'apprentissage choisie entre une méthode d'apprentissage automatique et une méthode d'apprentissage progressif.

Dans des modes de réalisation du procédé de l'invention :
- l'étape de détection de profils olfactifs comprend une étape de suppression d'une pollution COV de l'air ambient reçu par le dispositif, cette étape réalisée par un filtre du système avant l'entrée de l'air ambient dans le dispositif ;
- l'étape d'identification d'un profil olfactif comprend une étape de diminution de l'humidité et/ou de la température dans l'air ambiante reçu par le dispositif, cette étape étant réalisée par un condenseur du système ; et
- l'étape d'analyse des échantillons capturés par le dispositif comprend en outre une étape d'analyse de profils olfactifs dans les échantillons avant l'analyse réalisée par le dispositif, cette étape étant réalisée par un multiplexeur du système .

Dans des modes de réalisation du procédé de l'invention, l'étape de démarrage d'un cycle de mélangeage comprend une étape d'introduction, dans le moyen de mélangeage, des matières premières nécessaires à la réalisation de la production du mélange.

Dans des modes de réalisation du procédé de l'invention, l'étape de démarrage d'un cycle de mélangeage comprend une étape d'introduction, dans le moyen de mélangeage, d'un ou des masterbatchs.

Dans des modes de réalisation du procédé de l'invention, le procédé comprend en outre une étape de simulation d'un nombre de mélanges permettant de réaliser au moins un mélange caoutchouteux ayant des propriétés physico-chimiques prédéterminées.

L'invention concerne aussi un système de fabrication de produits caoutchouteux qui réalise les procédés de contrôle de fabrication de produits caoutchouteux divulgués, caractérisé en ce que le système comprend :
- une installation de production qui met en œuvre des étapes successives de mélangeage, l'installation de production comprenant au moins un moyen de mélangeage duquel un ou des mélanges caoutchouteux sortent;
   - un dispositif de détection d'odeur qui capture d'un air ambient autour de l'installation de production pour obtenir au moins un échantillon gazeux pendant le cycle de mélangeage ; et
- un sous-système de contrôle qui emploie un modèle de profils olfactifs à partir des caractéristiques physico-chimiques des mélanges caoutchouteux reconnus par résonance plasmonique de surface (SPR) ;
   de sorte que le dispositif reconnaît la présence des composés organiques volatiles odorants (COV) présents dans l'air ambiant capturé en mesurant sa concentration par résonance plasmonique de surface (SPR); et
   de sorte que le modèle de profils olfactifs apprend les propriétés physico-chimiques des COV associées avec les mélanges sortant du moyen de mélangeage pendant les cycles de production de produits caoutchouteux réalisés par l'installation de production.

Dans des modes de réalisation du système de l'invention, le sous-système comprend un ou des capteurs qui se déclenchent lorsque le modèle de profils olfactifs sortant indique un décalage entre les propriétés physico-chimiques du mélange en cours de production à l'installation de production et les propriétés physico-chimiques attendues. Dans des tels modes de réalisation du système de l'invention, le sous-système ajuste le fonctionnement de l'installation de production en réponse aux capteurs déclenchés pour obtenir un niveau de collant du mélange à partir d'une empreinte SPR fournie par le dispositif.

Dans des modes de réalisation du système de l'invention, le moyen de mélangeage est choisi parmi une ou des extrudeuses et/ou un ou des mélangeurs internes.

D'autres aspects de l'invention vont devenir évidents grâce à la description détaillée suivante.

### Brève description des dessins

La nature et les divers avantages de l'invention vont devenir plus évidents à la lecture de la description détaillée qui suit, conjointement avec les dessins annexés, sur lesquels les mêmes numéros de référence désignent partout des parties identiques, et dans lesquels :
**[****Fig 1****]** La figure 1 représente un ensemble des groupes chimiques obtenus à partir d'un caoutchouc chauffé.
**[****Fig 2****]** La figure 2 représente un mode de réalisation d'un système de fabrication de produits caoutchouteux de l'invention.
**[****Fig 3****]** La figure 3 représente un mode de réalisation du système de la figure 2.
**[****Fig 4****]** La figure 4 représente un mode de réalisation d'un procédé de contrôle de fabrication de produits caoutchouteux réalisé par le système de l'invention.

### Description détaillée

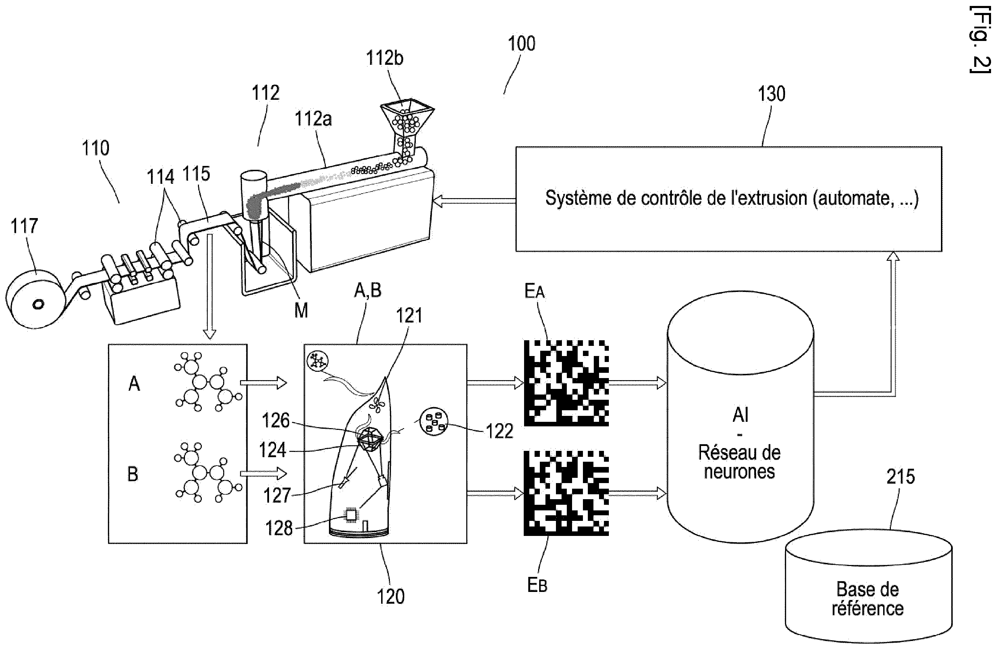

En se référant maintenant aux figures, sur lesquelles les mêmes numéros identifient des éléments identiques, la figure 2 représente un système de fabrication de produits caoutchouteux (ou « système ») 100 de l'invention. Le système 100 réalise un procédé de contrôle de fabrication de produits caoutchouteux en réponse à l'entrée des données obtenues par un outil de détection permettant une classification physico-chimique (aussi appelé un « nez électronique ») du système. Le système 100 est utilisable dans les installations où des produits caoutchouteux sont fabriqués (y compris des pneumatiques). Il est entendu que le système 100 peut fonctionner dans plusieurs environnements physiques sans connaissance de leurs paramètres en avance (par exemple, un arrangement initial d'une ligne de production de produits caoutchouteux de laquelle le système 100 fait partie).

Le système 100 comprend une installation de production (ou « installation ») 110 qui met en œuvre des étapes successives de mélangeage et de fin de ligne. Comme représenté dans la figure 2, l'installation de production 110 comprend au moins un moyen de mélangeage 112 qui met en œuvre des étapes successives de mélangeage d'un mélange caoutchouteux. A titre d'exemple, le moyen de mélangeage 112 peut comprendre au moins une extrudeuse 112a avec un châssis avec des parties communes assemblées, qui peuvent comprendre, sans limitation, un ensemble vis-fourreau (avec ou sans ses accessoires facultatifs de chauffage et de refroidissement ), un groupe d'entraînement (du réducteur et accouplement), un moteur principal, des dispositifs pour l'alimentation en matière (par exemple, des doseurs ou des trémies 112b), une armoire de pilotage qui réunit les variateurs des moteurs, des organes de démarrage et de sécurité, et des dispositifs de régulation, de commande, d'affichage et de mesure. L'extrudeuse 112a peut être une extrudeuse monovis, une extrudeuse bi-vis ou une extrudeuse à plusieurs vis.

Il est entendu que le moyen de mélangeage 112 peut incorporer, au lieu de l'extrudeuse 112a, un ou des mélangeurs internes connus (non représentés) qui réalisent la fabrication d'un mélange initial de matériaux élastomères avec une charge de noir de carbone et/ou de silice. Par « mélangeur interne », on entend une machine constituée d'un pilon et de deux demi-cuves (ou « cuves »), chacune contenant un rotor avec une ou plusieurs pâles (par exemple, une machine de type Banbury ou Intermix pour polymères). Dans un autre exemple, le moyen de mélangeage 112 peut incorporer au moins un mélangeur externe automatisé (également appelé « mélangeur à cylindres » ou « outil à cylindres ») dans lequel est ensuite transféré ce mélange travaille en le faisant encore circuler entre deux cylindres de manière à le transformer en une nappe continue. Des produits de vulcanisation (incluant, sans s'y limiter, le soufre) peuvent être ajoutés au mélange plus tard dans un cycle de mélangeage pour obtenir le mélange final à usage commercial.

A titre d'exemple, l'installation de production 110 comprend en outre un ou des outils à cylindres 114 qui mettent en forme d'une bande 115 le mélange M sortant de l'extrudeuse 112a. La bande 115 passe vers une ou des outils à cylindres 114 pour la mettre en forme continue avec une largeur prédéterminée et pour réaliser le refroidissement du mélange fabriqué. Il est entendu que chaque outil à cylindres peut comprendre des moyens de refroidissement internes comme connus par l'homme du métier.

L'installation de production 110 peut comprendre un dispositif de coupe ou de mise en forme de la matière extrudée. Par exemple, la bande 115 continue formée peut être enroulée en rouleaux (représentés, à titre d'exemple, par le rouleau 117 de la figure 2) pour faciliter le stockage du mélange produit.

Il est entendu que la configuration de l'installation de production 110 est donnée à titre d'exemple et que d'autres configurations peuvent être incorporées dans le système 100.

En se référant encore à la figure 2, le système 100 comprend aussi un dispositif de détection d'odeur (ou « dispositif ») 120 visant à reconnaître la présence d'un composé cible en mesurant sa concentration par résonance plasmonique de surface (« SPR »). Le dispositif 120, qui reçoit un air ambient autour de l'installation de production 110, peut comprendre un ventilateur 121 (ou un dispositif d'aspiration équivalent) qui aspire l'air ambient et le fait entrer dans le dispositif. Le flux d'air ambient peut être réglé par un moyen d'aération pouvant être sélectivement garder et évacuer l'air ambient (par exemple, une vanne pouvant être sélectivement ouvert et fermé).

Le dispositif 120 comprend aussi un ou des capteurs 122 qui détectent la présence des composés organiques volatiles odorants (ou « COV ») présents dans l'air ambient autour de l'installation de production 110. Les capteurs 122 incorporent une partie sensible qui interagit avec les COV (par exemple, des COV émis par le mélange M en cours de production à l'installation de production 110 et représentés par les molécules A et B dans la figure 2). Chaque capteur 122 peut détecter les composés d'une famille prédéterminée de composés. Comme entendu par l'homme du métier, cette partie sensible peut se constituer de matériaux non biologiques (tels que des oxydes métalliques semi-conducteurs (ou « MOS ») et des polymères semi-conducteurs) ou des molécules organiques (par exemple, des peptides).

Le dispositif 120 comprend en outre un film mince, et particulièrement une couche métallique 124, présentant une surface qui reste au contact avec l'air ambient entrant dans le dispositif. La couche métallique 124 comprend une couche d'un métal connu pour réaliser l'effet SPR (par exemple, l'or ou l'argent). Les capteurs 122 sont disposés sur cette couche métallique 124 dans un positionnement prédéterminé.

Le dispositif 120 comprend aussi un prisme 126 ayant une face d'entrée qui permet l'entrée de lumière, une face de sortie qui permet la sortie de lumière et une face de support sur laquelle la couche métallique est disposée. Dans un procédé réalisant l'effet SPR, un dispositif d'éclairage 127 du dispositif 120 émit une lumière collimatée au travers de la face d'entrée du prisme 126 jusqu'à une surface de la couche métallique 124, cette surface présentant une réflectivité connue. Le dispositif d'éclairage 127 peut être choisi parmi les dispositifs d'éclairage disponibles dans le commerce, y compris, sans limitation, des lumières du type LED (ou « light emitting diode » en anglais).

Le dispositif d'éclairage 127, étant sensible à l'indice de réfraction de l'air ambient présente dans le dispositif 120, produit une résonance de plasmons sur la surface de la couche métallique 124. Cette résonance, étant sensible à l'indice de réfraction de l'air ambient présente dans le dispositif 120, diminue la réflectivité de la couche métallique 124 pour qu'elle varie à proximité de chaque capteur 122. Le dispositif 120 peut être choisi parmi des dispositifs de détection d'odeur (ou « nez électroniques ») disponibles dans le commerce (par exemple, du type « NeOse » offert par la société Aryballe, mais il est entendu que d'autres dispositifs équivalents peuvent être utilisés).

Le dispositif 120 incorpore au moins un processeur 128 qui est configuré à détecter les COV présents dans l'air ambient et capturés par les capteurs 122. Le terme "processeur" (ou, alternativement, le terme "circuit logique programmable") désigne un ou plusieurs dispositifs capables de traiter et d'analyser des données et comprenant un ou plusieurs logiciels pour leur traitement (par exemple, un ou plusieurs circuits intégrés connus par l'homme de métier comme étant inclus dans un ordinateur, un ou plusieurs contrôleurs, un ou plusieurs microcontrôleurs, un ou plusieurs micro-ordinateurs, un ou plusieurs automates programmables (ou « PLC »), un ou plusieurs circuits intégrés spécifiques à une application, un ou plusieurs réseaux de neurones, et/ou un ou plusieurs autres circuits programmables équivalents connus). Le processeur 128 comprend un ou des logiciels pour le traitement des composés volatiles capturés par le dispositif 120 (et les données correspondantes obtenues) ainsi qu'un ou des logiciels pour l'identification et la classification des profils olfactifs permettant l'identifications des COV dans le cours de production des mélanges caoutchouteux. Le processeur 128 comprend aussi un ou des logiciels pour le traitement des sous-systèmes associés avec le système 100 (et les données correspondantes obtenues) ainsi qu'un ou des logiciels pour l'identification des variances et l'identification de leurs sources pour les corriger.

Pour bien gérer la reconnaissance de l'odeur entrant dans le dispositif 120, il faut identifier, dans les COV présents dans l'air ambient autour de l'installation de production 110, l'odeur caractérisant le ou les produits caoutchouteux en cours de production. Particulièrement, l'identification de profils olfactifs correspondant aux plusieurs composés volatils est pertinente pour déterminer des corrélations entre une propriété physico-chimique liée au caoutchouc (par exemple, le collant) et les évolutions des COV provenant des mélanges de caoutchouc. L'identification de profils olfactifs peut être réalisée d'une manière incorporant la construction d'un ou des modèles associés avec les aspects chimiques SPR et les aspects olfactifs d'un ou des composés cibles (aussi appelé « le modèle de profils olfactifs »). Un modèle de profils olfactifs peut être utilisé en apprenant les propriétés physico-chimiques associées avec les mélanges caoutchouteux pendant les cycles de production de produits caoutchouteux.

Le terme "composé cible" (dans le singulier ou le pluriel) est utilisé ici pour faire référence à un composé associé avec un produit caoutchouteux (y compris le mélange M) en cours de production dans l'environnement physique du système 100 et qui est identifié en fonction de données SPR obtenues par le dispositif 120. Afin de créer une "boîte noire" liée aux mélanges caoutchouteux et leurs profils olfactifs, les paramètres des mélanges caoutchouteux différents peuvent être utilisés pour former un ou plusieurs modèles de profils olfactifs. Ces données accumulées dans la boîte noire peuvent être utilisées pour prendre des décisions concernant le contrôle des paramètres de l'installation de production 110 du système 100 en examinant les profils odorants des mélanges caoutchouteux sortant de l'installation, les profils odorants actuels disponibles, les profils odorants des mélanges similaires, et/ou le temps passé à détecter les COV particuliers dans un cycle de mélangeage.

En se référant encore à la figure 2, un mode de réalisation d'intelligence artificielle peut être employé par le système 100 pour construire au moins un modèle de profils olfactifs. Dans des modes de réalisation, le processeur 128 (seul ou en combinaison avec un ou d'autres processeurs) peut configurer le système 100 (et notamment le dispositif 120) sur un ou plusieurs propriétés physico-chimiques enregistrées dans le modèle de profils olfactifs. Le processeur 128 peut également se référer à une référence pour effectuer une détermination finale du ou des propriétés physico-chimiques attendues. La référence peut comprendre au moins une base de référence incorporant, par exemple, un tableau de COV de mélanges caoutchouteux variés (y compris, sans limitation, des propriétés physico-chimiques correspondant aux COV à un temps précis dans le cours d'un cycle de mélangeage d'un mélange caoutchouteux). Le processeur 128 peut comparer des propriétés physico-chimiques du mélange M sortant du moyen de mélangeage 112 et le profil olfactif du mélange identifié, y compris les COV dévoilés par l'effet SPR, de sorte que le système 100 puisse ajuster l'installation de production 110 où les propriétés souhaitées du mélange M ne sont pas atteintes (par exemple, en envoyant une commande d'ajustement de l'installation de production 110 pour prévoir l'atteint des propriétés souhaitées du mélange). Le processeur 128 peut récupérer les propriétés physico-chimiques de l'air ambient capturé dans le dispositif 120 qui correspondent le plus étroitement aux propriétés reconnues pour configurer le dispositif. Une référence de l'air ambient peut inclure aussi des mesures COV correspondant à une pluralité de profils olfactifs connus (voir, à titre d'exemple, la figure 1).

Les données correspondantes à l'air ambient capturée par le dispositif 120 sont transférées et stockées dans la mémoire du processeur 128. Le processeur 128, qui exécute les instructions d'un module de traitement de données du processeur, analyse les données pour déterminer un ou plusieurs profils olfactifs correspondant au mélange de caoutchouc en cours de production à l'installation de production 110. Les profils olfactifs sont généralement les indicateurs des caractéristiques physico-chimiques du mélange en cours de production. La détection de différents groupes chimiques, comme les COV, permet de discriminer les émissions du mélange M. Le processeur 128 peut détecter des changements dans les propriétés du mélange M pour identifier au moins un composé volatil associé. D'autres caractéristiques du mélange M peuvent également être déterminés.

Les données capturées peuvent être appliquées à un déterminant de propriétés physico-chimiques, y compris les COV, qui peut exploiter un ou plusieurs modèles d'apprentissage machine pour générer les composés cible. Bien que les incarnations soient décrites ici en ce qui concerne l'utilisation des réseaux neuronaux (et plus particulièrement des réseaux neuronaux convolutifs, ou « convolutional neural network » en anglais or « CNN ») comme modèle d'apprentissage machine, d'autres types de modèles d'apprentissage machine peuvent être utilisés. Ceux-ci incluent, sans limitation, les modèles utilisant la régression linéaire, la régression logistique, les arbres de décision, les machines à vecteurs de support, les Bayes naïfs, le voisin le plus proche (knn), K signifie regroupement, forêt aléatoire, les algorithmes de réduction de la dimensionnalité, les algoïsmes à gradient, les réseaux de neurones (par exemple, les auto-encodeurs, les CNN, les RNN, les perceptrons, la mémoire logarithmique à court terme (LSTM), Hopfield, Boltzmann, la croyance profonde, la déconvolution, la confrontation générative (GAN), etc. ) et leurs compléments et équivalents. Le ou les CNNs peuvent être formés avec des données de vérité de terrain (ou « ground truth » en anglais) qui sont représentées dans une référence des COV créée pendant un procédé de contrôle de fabrication de produits caoutchouteux réalisé par le système 100.

Dans un mode de réalisation, le système 100 pourrait être optimisé par le principe d'apprentissage progressif (ou « shaping » en anglais). L'apprentissage progressif permet l'apprentissage de nouvelles tâches par la réutilisation de caractéristiques apprises dans des tâches antérieures et l'utilisation des caractéristiques en cours d'apprentissage. À un moment donné, les caractéristiques apprises être utilisées pour effectuer des tâches hors ligne qui peuvent être non supervisées, supervisées ou semi-supervisées. Le modèle, ayant déjà l'expérience de la politique à adopter dans un problème du même type (par exemple, des COV attribués à un mélange caoutchouteux collant), a une progression plus rapide que la performance obtenue par un apprentissage en partant de zéro. Pour l'invention divulguée, l'entrainement d'un réseau de neurones dans le but d'identifier les facteurs primaires de la SPR permettant ainsi l'estimation d'une propriété physico-chimique (par exemple, le collant, le vieillissement, la pollution, etc.), peut nécessiter une quantité énorme de données pour obtenir le résultat escompté. C'est pour cela que l'apprentissage progressif peut être choisi à l'optimisation du temps d'apprentissage.

Le système 100 de l'invention utilise donc l'effet SPR afin de fournir une ou des empreintes numériques (ou « empreintes SPR » ou « empreintes ») des COV capturés. A titre d'exemple, les empreintes E_{A} et E_{B} de la figure 2 représentent les COV émis par le mélange M.

En se référant encore à la figure 2, le système 100 comprend en outre un sous-système de contrôle (ou « sous-système ») 130. Le sous-système 130 emploie le modèle de profils olfactifs sortant du réseau neuronal pour contrôler le fonctionnement de l'installation de production 110 en réponse à la présence des composés cibles dans l'air ambient de l'installation. Les capteurs du sous-système 130 peuvent déclencher lorsque le modèle de profils olfactifs sortant indique un décalage entre les propriétés physico-chimiques du mélange M en cours de production à l'installation de production 110 (représenté par l'empreinte SPR du mélange fourni par le dispositif 120) et les propriétés physico-chimiques attendues. Le(s) capteurs 122 du dispositif 120 peuvent détecter la présence des composés cibles dans l'air ambient autour de l'installation de production 110, ce qui déclenche le sous-système 130 de changer la vitesse de rotation des vis dans l'extrudeuse 112a. A titre d'exemple, les capteurs 122 du dispositif 120 peuvent détecter la présence des composés cibles à un niveau indiquant un mélange trop collant, permettant une modification des matières premières entrant à l'extrudeuse 112a. Dans un autre exemple, la détection de l'air ambient par le dispositif 120 peut signaler un écoulement nécessitant un changement de vis (par exemple, les vis pouvant être choisies parmi des profils co-rotatifs interpénétrés avec profils conjugués, des profils conjugués ou non, des vis parallèles ou coniques, des profils mono-filets ou multi-filets, et des vis modulaire ou non).

Dans certains modes de réalisation, le sous-système 130 comprend un ou des capteurs (non représentés) qui déclenchent lorsqu'une image de l'installation de production 110, ensemble avec le modèle de profils olfactifs sortant, indique un décalage entre les propriétés physico-chimiques du mélange caoutchouteux en cours de production à l'installation de production 110 et les propriétés physico-chimiques attendues. Dans ce sens, un capteur du sous-système 130 peut comprendre une caméra, un appareil photo, un capteur optique et/ou d'autres types de détection équivalents.

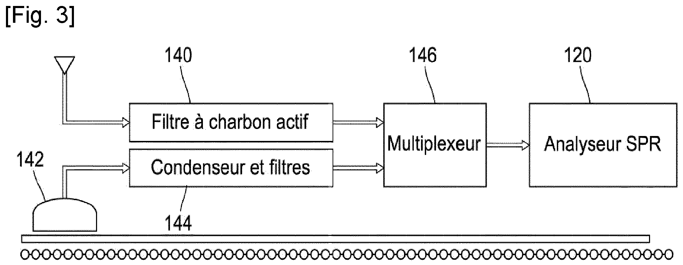

En se référant encore à la figure 2 et en outre à la figure 3, un autre mode de réalisation du système 100 est représenté de manière schématique. Dans ce mode de réalisation du système 100, le système 100 comprend l'installation de production 110 et le dispositif 120 comme décrits ci-dessus par rapport au mode de réalisation représenté dans la figure 2. Le système 100 comprend aussi au moins un filtre 140 pour supprimer une pollution COV de l'environnement autour de l'installation de production 110. Le filtre 140 permet d'appauvrir la pollution COV (en soufre, par exemple), permettant donc l'obtention d'une référence stable pour construire le modèle de profils olfactifs. Dans un mode de réalisation du filtre 140, le filtre comprend un filtre à charbon actif qui est bien connu comme un purificateur de l'air. Pour réaliser la captation des COV du ou des mélanges en cours de production, le système 100 comprend en outre une coupole de concentration de gaz 142 qui communique avec un condenseur 144 du système 100 (la coupole 142 est choisie parmi les coupoles disponibles dans le commerce, par exemple, du type offert par la sociéte SoluProTech). Le condenseur 144, qui est choisi parmi les condenseurs connus et disponibles dans le commerce, permet de diminuer l'humidité et la température de l'air ambient. Le système 100 comprend aussi un multiplexeur 146 permettant l'analyse de multiples composés cibles avant l'analyse SPR réalisée par le dispositif 120.

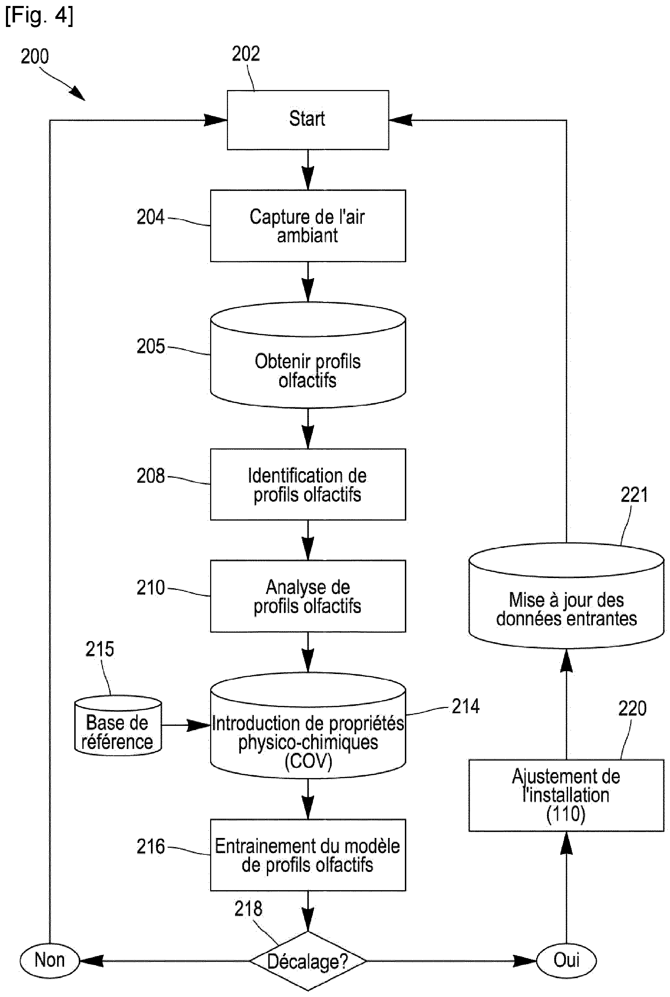

En se référant encore aux figures 2 et 3, et en outre à la figure 4, une description détaillée est donnée à titre d'exemple d'un procédé de contrôle de fabrication de produits caoutchouteux en réponse à l'entrée des données obtenues par le dispositif 120 (ou « procédé de contrôle de fabrication » ou « procédé ») 200 de l'invention réalisé par le système 100.

En lançant un procédé de contrôle de fabrication de produits caoutchouteux 200 de l'invention, le procédé comprend une étape de démarrage 202 d'un cycle de mélangeage à l'installation de production 110. Cette étape comprend une étape d'introduction, dans le moyen de mélangeage 112, des différentes matières premières nécessaires à la réalisation de la production de produits caoutchouteux ayant des propriétés souhaitées (voir la flèche A de la figure 2). Ces matières premières comprennent, sans limitation, un matériau élastomère (par exemple, un caoutchouc naturel, un élastomère synthétique et des combinaisons et équivalents de ceux-ci) et un ou plusieurs ingrédients, tels qu'un ou plusieurs agents de mise en œuvre, agents de protection et charges de renforcement. Les matières premières peuvent également comprendre un ou plusieurs autres ingrédients tels que le noir de carbone, la silice, les huiles, les résines et les agents de réticulation ou vulcanisation (par exemple, le soufre). Tous les ingrédients sont introduits dans des quantités variables en fonction des performances souhaitées des produits obtenus des procédés de mélangeage (par exemple, des pneumatiques).

Le cycle de mélangeage peut également se faire en démarrant le cycle avec un produit déjà mélangé mais ne contenant pas tous les ingrédients de la recette de mélange choisie (appelé « masterbatch »). Par exemple, les résines et les agents de vulcanisation ne sont pas présents dans le masterbatch. Dans ce cas, soit le masterbatch est récupéré chaud en provenance d'un mélangeur en amont de l'installation de production 110 (tel qu'un mélangeur interne ou un mélangeur externe), soit le masterbatch est froid car il a été fabriqué et conditionné plusieurs heures voire plusieurs jours au préalable.

Pendant cette étape, le moyen de mélangeage 112 est mis en œuvre (par exemple, dans les cas où le moyen de mélangeage comprend l'extrudeuse 112a, la ou les vis de l'extrudeuse sont mises en rotation pour provoquer un mouvement du mélange caoutchouteux vers l'aval de l'extrudeuse dès l'introduction des matières premières) (voir la flèche B de la figure 2). Il est entendu que le cycle de mélangeage peut être facilement adapté pour tous les modes de réalisation de l'installation de production 110 (par exemple, les modes de réalisation incorporant des mélangeurs internes au lieu de l'extrudeuse 112a). Pendant cette étape, les outils à cylindres 114 misent en forme la bande 115 du mélange M sortant du moyen de mélangeage 112, et la bande 115 continue formée est enroulée en rouleaux 117 pour faciliter le stockage du mélange.

Le procédé de contrôle de fabrication de l'invention comprend aussi une étape de capture 204 de l'air ambient autour de l'installation de production 110 pour obtenir un ou des échantillons gazeux pendant la production de la bande 115. Cette étape, étant réalisée par le dispositif 120 du système 100, peut être réalisée d'une manière itérative en fonction de nombre d'échantillons prévus pour alimenter le réseau neuronal. L'étape de capture met en œuvre un effet SPR utilisant un changement local de l'indice de réfraction du mélange caoutchouteux produit par l'installation de production 110.

Le procédé de contrôle de fabrication 200 de l'invention comprend en outre une étape de détection des profils olfactifs, y compris les COV, présents dans l'air ambient et capturés par les capteurs 122 du dispositif 120. Pendant cette étape, ces profils olfactifs détectés sont enregistrés dans une ou des bases de données 205. Des paramètres différents, tels que les caractéristiques des gaz (y compris la nature et la concentration des produits chimiques, les propriétés physico-chimiques des produits chimiques, par exemple, solubilité, pression de vapeur saturante et biodégradabilité), les conditions d'exploitation et les variations du taux d'humidité de l'environnement peuvent influencer la durée de cette étape.

Dans les modes de réalisation du système 100 incorporant un filtre 140, cette étape comprend une étape de suppression de la pollution COV de l'air ambient avant son entrée dans le dispositif 120.

Le procédé de contrôle de fabrication 200 de l'invention comprend en outre une étape d'identification 208 d'un profil olfactif pour identifier le mélange caoutchouteux en cours de production et son état de production. Cette étape est réalisée à partir d'au moins un échantillon de l'air ambient capturée par le dispositif 120. Les identifications peuvent être réalisées en fonction du nombre d'échantillons d'être capturé. Pendant cette étape, les données extraites sont traitées et normalisées par le dispositif 120 pour que les COV puissent être mesurés par l'effet SPR. Le dispositif 120 fournit une empreinte des COV présents dans chaque échantillon mesuré (voir, par exemple, les empreinte E_{A} et E_{B} représentées dans la figure 4). Cette empreinte « olfactive », étant une image du profil olfactif à l'instant de sa mesure, varie en fonction des traitements réalisés sur le mélange ou son évolution attendue pendant la réalisation d'un cycle de mélangeage.

Dans les modes de réalisation du système 100 incorporant le condenseur 144, cette étape comprend une étape de diminution de l'humidité et/ou de la température dans l'air ambiante capturée par le dispositif 120.

Dans un mode de réalisation, pendant l'étape d'identification 208 d'un profil olfactif, le dispositif 120 peut produire automatiquement plusieurs empreintes (par exemple, dans le cours d'une calibration chimique telle que décrite dans la publication FR3063543). Le signal représentatif de l'indice optique local d'un milieu gazeux est volontairement variable pour augmenter la robustesse du réseau neuronal et pour assurer son acuité.

Le procédé de contrôle de fabrication 200 de l'invention comprend en outre une étape d'analyse 210 des échantillons capturés par le dispositif 120. De l'empreinte fournie par le dispositif 120 et les données extraites représentées dedans, on extrait les caractéristiques qui alimentent un algorithme de classification des COV selon des propriétés physico-chimiques. Les caractéristiques mentionnées ici peuvent inclure, sans limitation, les caractéristiques des mélanges collants, des mélanges de-cohésifs, l'ajout d'eau aux mélanges et le vieillissement des mélanges.

Dans les modes de réalisation du système 100 incorporant le multiplexeur 146, cette étape comprend une étape d'analyse de multiples composés cibles dans chaque échantillon avant l'analyse réalisée par le dispositif 120.

Le procédé de contrôle de fabrication 200 de l'invention comprend en outre une étape de construction du modèle de profils olfactifs sur la base des propriétés physico-chimiques du mélange caoutchouteux identifié. Pour réaliser la construction du modèle de profils olfactifs, cette étape comprend une étape d'introduction 214 au réseau neuronal des propriétés physico-chimiques du mélange caoutchouteux identifié, cette étape étant réalisée par le système 100. Pendant cette étape, des propriétés physico-chimiques du mélange caoutchouteux identifié sont obtenues par le dispositif 120. Les propriétés physico-chimiques obtenues comprennent des données correspondantes à l'empreinte fournie par le dispositif 120. Ces données sont enregistrées (par exemple, dans une ou des bases de données 150 du système 100) (voir la figure 2), et elles sont mises à jour pendant la durée du procédé de contrôle de fabrication sur une base continue ou sur une base intermittente.

L'étape d'introduction 214 comprend une étape de création d'une base d'apprentissage (ou « base ») 215 des propriétés physico-chimiques qui est introduite dans le modèle de profils olfactifs. Les propriétés physico-chimiques correspondent aux profils olfactifs des échantillons capturés par les capteurs 122 du dispositif 120. Cette étape comprend une étape de création d'une référence des profils olfactifs cherchés dans l'air ambient d'être capturée par le dispositif 120. La référence des profils olfactifs qui est créée pendant cette étape comprend des profils olfactifs attendues correspondant aux profils olfactifs connus des mélanges caoutchouteux en cours de production à l'installation de production 110. Cette étape peut être réalisée en avance d'autres étapes du procédé pour alimenter le réseau neuronal les vrais profils olfactifs attendus en analysant l'air ambient capturée pendant plusieurs cycles de mélangeage.

La base d'apprentissage créée peut comprendre au moins une base de référence 160 déjà créée (par exemple, un tableau de COV de mélanges caoutchouteux variés discuté ci-dessus). La base peut inclure des paramètres correspondants à une pluralité de recettes de mélanges caoutchouteux connues (y compris des paramètres qui font partie de l'information générale). Dans des modes de réalisation du procédé 200, au moins une partie de la référence des profils olfactifs est créée par un ou des hommes du métier (par exemple, pendant un enregistrement des données capturées dans l'air ambiante d'autres installations produisant un mélange similaire au mélange M sortant de l'installation de production 110).

Le procédé de contrôle de fabrication 200 de l'invention comprend en outre une étape d'entrainement 216 du modèle de profils olfactifs. Pendant cette étape, une méthode d'apprentissage automatique prend en entrée les profils olfactifs obtenus du mélange caoutchouteux ainsi que les données de la base d'apprentissage créée. Après que le système 100 a obtenu les profils olfactifs du mélange caoutchouteux identifié, le processeur 128 peut récupérer ses propriétés physico-chimiques connus correspondant pour construire le modèle de profils olfactifs.

Dans un mode de réalisation, la méthode d'apprentissage automatique employée pendant l'étape d'entrainement 216 comprend une méthode d'apprentissage supervisé. La méthode d'apprentissage supervisé peut comprendre l'entrainement d'un ou des réseaux neuronaux comme discuté ci-dessus.

Ainsi, le modèle de profils olfactifs sortant sera l'identification des propriétés physico-chimiques du mélange caoutchouteux en cours de production à l'installation de production 110 (caractérisé, par exemple, par l'atteint des propriétés souhaitées du mélange à un temps prédéterminé). Ceci permet, par exemple, d'effectuer des actions préventives pour assurer l'atteint d'un mélange caoutchouteux ayant les propriétés souhaitées. A titre d'exemple, dans certains cas d'usage, une fois l'apprentissage réalisé, le système 100 peut estimer le collant produit à partir d'une empreinte SPR et d'autres données de base (par exemple, la température et l'humidité).

Dans des modes de réalisation du procédé de contrôle de fabrication 200 de l'invention, le procédé comprend en outre une étape de comparaison 218. Pendant cette étape, l'identification des propriétés physico-chimiques du mélange M sortant du moyen de mélangeage 112, ressorti du modèle de profils olfactifs, est comparé au profil olfactif du mélange identifié, de sorte que le système 100 puisse ajuster l'installation de production 110 où les propriétés souhaitées du mélange M ne sont pas atteintes. Dans ces cas, le procédé 200 comprend en outre une étape de commande 220 d'ajustement d'au moins un élément de l'installation de production 110. Pendant cette étape, la commande d'ajustement est envoyée au sous-système 130 pour gérer l'installation de production 110 (par exemple, pour déclencher les capteurs du sous-système 130 de sorte que la vitesse des vis de l'extrudeuse est ralentie).

Pendant cette étape, le système 100 met à jour les données entrantes (voir la base de données 221) pour aider le partage des informations sur les propriétés physico-chimiques des mélanges caoutchouteux à un site de production incorporant l'installation de production 110 (y compris des opérateurs du site incorporant l'installation de production 110). Le système 100 met en œuvre le procédé 200 de l'invention en utilisant des données de mesure des COV "stockées" et les données « mises à jour » pour préparer des rappels personnalisés à envoyer à l'installation de production 110 (ou à un site incorporant l'installation de production 110 ou à un ou des opérateurs du site incorporant l'installation de production 110) et pour prévoir l'atteint des propriétés souhaitées du mélange. Dans ce mode de réalisation, le système 100 peut proposer des ajustements de paramètres du cycle de mélangeage pour améliorer le mélange caoutchouteux sortant de l'installation de production 110.

Tel qu'utilisé ici, "opérateur" ou « utilisateur » se réfère à un seul opérateur ou à un groupe d'opérateurs. Un opérateur comprend, sans s'y limiter, un participant individuel dans une tâche d'un cycle de mélangeage réalisé par l'installation de production 110, un membre individuel d'une équipe ou d'un groupe qui participe à un cycle de mélangeage, une ou plusieurs machines associées à un individuel ou une équipe qui participe à au moins une tâche d'un cycle de mélangeage, une communauté numérique associée à un cycle de mélangeage et des combinaisons et équivalents de ceux-ci. L'opérateur peut être un spectateur qui assiste à un cycle de mélangeage, en totalité ou en partie, physiquement ou virtuellement (par exemple, en gérant à distance une opération prédéterminée en direct afin de voir l'installation de production 110 en temps réel). Tel qu'utilisé ici, "opérateur" peut également se référer à tout système ou appareil électronique configuré pour recevoir une entrée de commande et configuré pour envoyer automatiquement des données à au moins un autre opérateur.

Dans les modes de réalisation du procédé 200 de l'invention, le procédé 200 comprend en outre une étape de simulation d'un nombre de mélanges permettant de réaliser les produits caoutchouteux ayant des propriétés physico-chimiques prédéterminées. Chaque simulation fait l'objet par la suite d'une prédiction de profil olfactif d'un mélange caoutchouteux ayant des propriétés physico-chimiques attendues via un modèle de profils olfactifs comme décrit ci-dessus pour obtenir finalement une distribution de prévision de profils olfactifs pour les propriétés physico-chimiques identifiés.

Le système 100 de l'invention est donc basé sur un réseau de neurones dont le fondement est d'être entrainé sur un grand nombre de situations (par exemple, des échantillons de l'air ambiante) pour ensuite pouvoir décrire un nouveau mélange caoutchouteux qui lui serait présentée. D'une part, il faut expliquer au réseau neuronal ce qu'il doit reconnaitre, et puis les lui faire apprendre (« annotation »). D'autre part, il faut jauger la performance du réseau neuronal et proposer des échantillons pertinents pour éviter de tomber dans certains biais comme le surentrainement qui vont diminuer la performance du réseau. Ainsi, le principe du système 100 est d'effectuer simplement ce que l'on pourrait considérer comme une différence entre les éléments marqueurs de l'air ambiante et les éléments mesurés. Les effets de la pollution environnementale sont supprimés mathématiquement.

Il est envisageable qu'une ou des étapes du procédé puissent être réalisées de manière itérative.

### EXEMPLE

Grâce à des données de référence (ici le collant et la modélisation SPR associée), un réseau de neurones déjà entrainé à minima peut donc déterminer le niveau de collant en fonction des données SPR. En considérant le niveau de collant des mélanges dans les ateliers de fabrication, les propriétés d'une composition des mélanges influencent ce niveau, notamment :
- Le ratio du taux de charge renforçante et le taux de plastifiant, ce ratio indiquant un mélange plus collant en fonction de l'abaissement du ratio.
- Le ratio de résine dans le mélange, ce ratio indiquant un mélange plus collant en fonction de l'augmentation du ratio.
- La fraction volumique d'élastomère qui indique un mélange plus collant en fonction d'abaissement de la fraction volumique.

On considère aussi la caractéristique de l'élastomère qui peut entrer dans la composante du collant des mélanges. Dans cet exemple, on considère un Mooney ML1+4 100°C d'élastomère sec <50points, ou la présence d'isoprène. Le Mooney, également connu sous les noms de viscosité ou plasticité, caractérise, d'une manière connue, des substances solides. On utilise un consistomètre oscillant tel que décrit dans la norme ASTM D1646 standard (1999). Cette mesure de plasticité est effectuée selon le principe suivant: l'échantillon analysé à l'état brut (à savoir, avant cuisson) est moulée (formée) dans une enceinte cylindrique chauffée à une température donnée (par exemple 35°C ou. 100°C). Après une minute de préchauffage, le rotor tourne au sein de l'éprouvette à 2 tours/minute et le couple utile pour entretenir ce mouvement est mesuré pendant 4 minutes de rotation. La viscosité Mooney (ML 1 + 4) est exprimée en "unité Mooney"(avec 1UM=0,83 Nm) et correspond à la valeur obtenue à la fin des 4 minutes
Le terme « collant » définit une propriété d'un mélange de caoutchouc qui varie suivant les formulations des mélanges. Un mélange très collant n'est pas forcément un mélange « extrême » sur un des critères, mais c'est l'association de tous les critères qui donne son collant et donc sa difficulté de réalisation. En multipliant ces critères, un « indice de collant » est donc trouvé et représenté par le tableau en dessous :

**[Tableau.1]**

| **1 - Ratio Noir/Plastifiant** | | | | | | | | | | | |
|---|---|---|---|---|---|---|---|---|---|---|---|
| **Noir/Plastifiant indice** | **150.38** | **140.30** | **130.23** | **120.15** | **110.08** | **100.00** | **89.92** | **79.85** | **69.77** | **59.70** | **49.62** |
| **Valeur de sévérité** | **1** | **1.10** | **1.20** | **1.30** | **1.40** | **1.50** | **1.60** | **1.70** | **1.80** | **1.90** | **2.00** |

| **2 - Présence Résine** | | | | | | | | | | | |
|---|---|---|---|---|---|---|---|---|---|---|---|
| **Résine tackifiante indice** | **0.00** | **20.00** | **40.00** | **60.00** | **80.00** | **100.00** | **120.00** | **140.00** | **160.00** | **180.00** | **200.00** |
| **Valeur de sévérité** | **1** | **1.10** | **1.20** | **1.30** | **1.40** | **1.50** | **1.60** | **1.70** | **1.80** | **1.90** | **2.00** |

| **3 - Cohésion Mélange** | | | | | | | | | | | |
|---|---|---|---|---|---|---|---|---|---|---|---|
| **Fraction volumique d'élastomère indice** | **143** | **134** | **126** | **117** | **109** | **100** | **91** | **83** | **74** | **66** | **57** |
| **Valeur de sévérité** | **1** | **1.10** | **1.20** | **1.30** | **1.40** | **1.50** | **1.60** | **1.70** | **1.80** | **1.90** | **2.00** |

De façon empirique et suivant différents critères de faisabilité industrielle, un bilan est réalisé sur la faisabilité industrielle de plusieurs mélanges. La limite de faisabilité est établie pour des indices de collant au-dessus de 3.5 et 4.5 points.

### Exemples de mélanges :

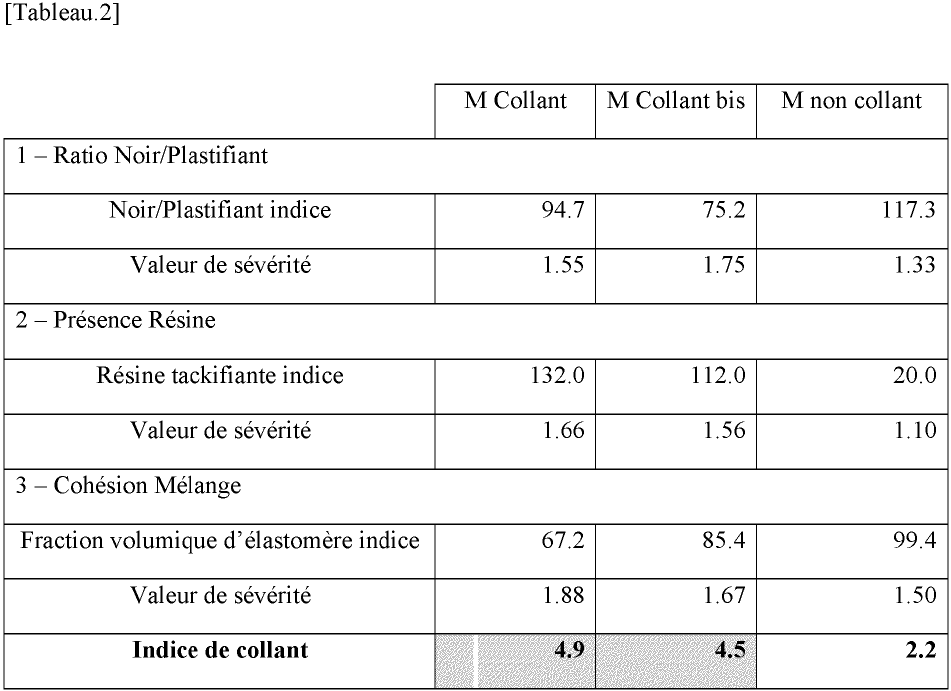

La mesure du collant d'un mélange caoutchouteux est indispensable lors de la fabrication de certains produits caoutchouteux. A titre d'exemple, pour des produits semi-finis fabriqués dans le domaine de production de pneumatiques, le collant détermine la solidité de l'assemblage de l'enveloppe et la cohésion des produits lors de la conformation.

Grâce au système 100 et son utilisation de l'effet SPR par le dispositif 120, l'invention divulguée est non seulement très performante, mais aussi souple et adaptable au cas par cas si besoin ou si les conditions opératoires devaient évoluer. Le système 100 est donc adaptée pour des produits caoutchouteux composés d'une variété de mélanges de caoutchouc. De ce fait, l'invention prend en compte la qualité des paramètres mesurés et analysés pour assurer la qualité des produits caoutchouteux obtenus.

Il est prévu d'incorporer d'autres dispositifs du type nez électronique dans les procédés de fabrication des produits caoutchouteux. A titre d'exemple, un nez électronique employé peut utiliser une technologie à base de la chromatographie gazeuse, à la base des cellules électrochimiques (par exemple, pour l'analyse de gaz cibles comme NH₃, H₂S, et CO₂), à la base de la détection à photo-ionisation (PID), et à la base de l'interférométrie (par exemple, l'interférométrie Mach Zehnder employée dans les cas où une sensibilisation réduite au soufre et aux autres composés agressifs est souhaitée).

Un cycle du procédé de contrôle de fabrication de l'invention peut être fait par la commande du PLC et peut inclure des pré-programmations des informations de gestion. Par exemple, un réglage de procédé peut être associé avec les propriétés souhaitées d'un mélange produit par l'installation de production 110, y compris les propriétés du moyen de mélangeage 112, les propriétés des matières premières entrant dans le moyen de mélangeage et les propriétés du mélange sortant du moyen de mélangeage.

Dans des modes de réalisation de l'invention, le système 100 (et/ou un site incorporant le système 100) peut recevoir des commandes vocales ou d'autres données audio représentant, pour exemple, une démarche ou un arrêt de capture d'échantillons de l'air ambiante entrant dans le dispositif 120. La demande peut inclure une demande pour l'état actuel d'un cycle de mélangeage réalisé par l'installation de production 110. Une réponse générée peut être représentée de manière audible, visuelle, tactile (par exemple, en utilisant une interface haptique), virtuelle et/ou augmentée.

Pour toutes les réalisations du système 100, un système de surveillance pourrait être mis en place. Au moins une partie du système de surveillance peut être fournie dans un dispositif portable tel qu'un dispositif de réseau mobile (par exemple, un téléphone mobile, un ordinateur portable, un ou des dispositifs portables connectés au réseau (y compris des dispositifs « réalité augmentée » et/ou « réalité virtuelle », des vêtements portables connectés au réseau et/ou toutes combinaisons et/ou tous équivalents).

Le système 100 permet une mesure en continue des mélanges caoutchouteux issus des cycles de mélangeages et un contrôle non-destructif qui supprime l'effet opérateur. Le système 100 de l'invention rend la mesure des caractéristiques physico-chimiques plus objective dans le sens qu'il évite la prise d'échantillons des caoutchoucs et rend une mesure répétable.

Les termes « au moins un(e) » et « un(e) ou plusieurs » sont utilisés de manière interchangeable. Les gammes qui sont présentées comme se situant « entre a et b » englobent les valeurs « a » et « b ».

Bien que des modes de réalisation particuliers de l'appareil révélé aient été illustrés et décrits, on comprendra que divers changements, additions et modifications peuvent être pratiqués sans s'écarter de l'esprit ni de la portée du présent exposé. Par conséquent, aucune limitation ne devrait être imposée sur la portée de l'invention décrite à l'exception de celles exposées dans les revendications annexées.

## Revendications

1. Un procédé (200) de contrôle de fabrication de produits caoutchouteux réalisé par un système (100) de fabrication de produits caoutchouteux comprenant une installation de production (110) ayant au moins un moyen de mélangeage (112) qui met en œuvre des étapes successives de mélangeage d'un mélange caoutchouteux, **caractérisé en ce que** le procédé comprend les étapes suivantes :
- une étape de démarrage (202) d'un cycle de mélangeage réalisé à l'installation de production (110);
- une étape de capture (204) d'air ambient autour de l'installation de production (110) pour obtenir au moins un échantillon gazeux pendant le cycle de mélangeage, cette étape étant réalisée par un dispositif (120) de détection d'odeur du système (100) qui reconnaît la présence des composés organiques volatiles odorants (COV) présents dans l'air ambiant reçu dans le dispositif (120) en mesurant sa concentration par résonance plasmonique de surface (SPR);
- une étape de détection de profils olfactifs présents dans l'air ambient et capturés par le dispositif (120) ;
- une étape d'identification (208) d'un profil olfactif pour identifier un mélange (M) sortant du moyen de mélangeage (112) et son état de production, cette étape étant réalisée à partir d'au moins un échantillon de l'air ambient capturée par le dispositif (120);
- une étape d'analyse (210) des échantillons capturés par le dispositif (120) à partir d'une ou des empreintes SPR (E_{A}, E_{B}) fournies par le dispositif (120) et les données extraites représentées dedans;
- une étape de construction d'au moins un modèle de profils olfactifs à partir des propriétés physico-chimiques du mélange caoutchouteux identifié, cette étape comprenant une étape d'introduction (214) à un réseau neuronal des propriétés physico-chimiques du mélange caoutchouteux identifié, cette étape étant réalisée par le système (100) ; et
- une étape d'entrainement (216) du modèle de profils olfactifs à partir des profils olfactifs du mélange caoutchouteux identifié;
de sorte que le modèle de profils olfactifs sortant sera l'identification des propriétés physico-chimiques du mélange caoutchouteux en cours de production à l'installation de production (110).

2. Le procédé (200) de la revendication 1, dans lequel l'étape de construction du modèle de profils olfactifs comprend une étape de création d'une référence des profils olfactifs cherchés dans l'air ambient d'être capturé par le dispositif (120).

3. Le procédé (200) de la revendication 2, dans lequel l'étape d'introduction (214) de l'étape de construction du modèle de profils olfactifs comprend une étape de création d'une base d'apprentissage (215) des propriétés physico-chimiques qui est introduite dans le modèle de profils olfactifs.

4. Le procédé (200) de l'une quelconque des revendications 1 à 3, comprenant en outre une étape de comparaison (218) pendant laquelle l'identification des propriétés physico-chimiques du mélange (M) sortant du moyen de mélangeage (112), ressorti du modèle de profils olfactifs, est comparé au profil olfactif du mélange identifié, de sorte que le système (100) puisse ajuster l'installation de production (110) où les propriétés souhaitées du mélange (M) ne sont pas atteintes.

5. Le procédé (200) de la revendication 4, comprenant en outre une étape de commande (220) d'ajustement de l'installation de production (110) pour prévoir l'atteinte des propriétés souhaitées du mélange (M) sortant du moyen de mélangeage (112).

6. Le procédé (200) de la revendication 5, dans lequel l'étape de commande (220) d'ajustement de l'installation de production (110) comprend une étape de détermination d'un niveau de collant du mélange (M) à partir d'une empreinte SPR fournie par le dispositif (120).

7. Le procédé (200) de l'une quelconque des revendications 1 à 6, dans lequel, pendant l'étape d'entrainement (216) du modèle de profils olfactifs, le système (100) emploie une méthode d'apprentissage choisie entre une méthode d'apprentissage automatique et une méthode d'apprentissage progressif.

8. Le procédé (200) de l'une quelconque des revendications 1 à 7, dans lequel :
- l'étape de détection de profils olfactifs comprend une étape de suppression d'une pollution COV de l'air ambient reçu par le dispositif (120), cette étape réalisée par un filtre (140) du système (100) avant l'entrée de l'air ambient dans le dispositif (120) ;
- l'étape d'identification (208) d'un profil olfactif comprend une étape de diminution de l'humidité et/ou de la température dans l'air ambiant reçu par le dispositif (120), cette étape étant réalisée par un condenseur (144) du système (100) ; et
- l'étape d'analyse (210) des échantillons capturés par le dispositif (120) comprend en outre une étape d'analyse de profils olfactifs dans les échantillons avant l'analyse réalisée par le dispositif (120), cette étape étant réalisée par un multiplexeur (146) du système (100).

9. Le procédé (200) de l'une quelconque des revendications 1 à 8, dans lequel l'étape de démarrage (202) d'un cycle de mélangeage comprend une étape d'introduction, dans le moyen de mélangeage (112), des matières premières nécessaires à la réalisation de la production du mélange (M).

10. Le procédé (200) de l'une quelconque des revendications 1 à 8, dans lequel l'étape de démarrage (202) d'un cycle de mélangeage comprend une étape d'introduction, dans le moyen de mélangeage (112), d'un ou des masterbatchs.

11. Le procédé (200) de l'une quelconque des revendications 1 à 10, comprenant en outre une étape de simulation d'un nombre de mélanges permettant de réaliser au moins un mélange (M) caoutchouteux ayant des propriétés physico-chimiques prédéterminées.

12. Un système (100) de fabrication de produits caoutchouteux qui réalise un procédé (200) de contrôle de fabrication de produits caoutchouteux, **caractérisé en ce que** le système (100) comprend :
- une installation de production (110) qui met en œuvre des étapes successives de mélangeage, l'installation de production comprenant au moins un moyen de mélangeage (112) duquel un ou des mélanges (M) caoutchouteux sortent;
- un dispositif (120) de détection d'odeur qui capture d'un air ambient autour de l'installation de production (110) pour obtenir au moins un échantillon gazeux pendant le cycle de mélangeage ; et
- un sous-système de contrôle (130) qui emploie un modèle de profils olfactifs à partir des caractéristiques physico-chimiques des mélanges caoutchouteux reconnus par résonance plasmonique de surface (SPR) ;
de sorte que le dispositif (120) reconnaît la présence des composés organiques volatiles odorants (COV) présents dans l'air ambiant capturé en mesurant sa concentration par résonance plasmonique de surface (SPR); et
de sorte que le modèle de profils olfactifs apprend les propriétés physico-chimiques des COV associées avec les mélanges (M) sortant du moyen de mélangeage (112) pendant les cycles de production de produits caoutchouteux réalisés par l'installation de production (110).

13. Le système (100) de la revendication 12, dans lequel le sous-système (130) comprend un ou des capteurs qui se déclenchent lorsque le modèle de profils olfactifs sortant indique un décalage entre les propriétés physico-chimiques du mélange (M) en cours de production à l'installation de production (110) et les propriétés physico-chimiques attendues.

14. Le système (100) de la revendication 13, dans lequel le sous-système (130) ajuste le fonctionnement de l'installation de production (110) en réponse aux capteurs déclenchés pour obtenir un niveau de collant du mélange (M) à partir d'une empreinte SPR fournie par le dispositif (120).

15. Le système (100) de l'une quelconque des revendications 12 à 14, dans lequel le moyen de mélangeage (112) est choisi parmi une ou des extrudeuses et/ou un ou des mélangeurs internes.

## Patentansprüche

1. Verfahren (200) zur Steuerung der Herstellung von Kautschukprodukten, das durch ein System (100) zur Herstellung von Kautschukprodukten durchgeführt wird, das eine Produktionsanlage (110) beinhaltet, die mindestens ein Mischmittel (112) aufweist, das aufeinanderfolgende Schritte des Mischens einer Kautschukmischung umsetzt, **dadurch gekennzeichnet, dass** das Verfahren die folgenden Schritte beinhaltet:
- einen Schritt des Startens (202) eines Mischzyklus, der an der Produktionsanlage (110) durchgeführt wird;
- einen Schritt des Erfassens (204) von Umgebungsluft, die die Produktionsanlage (110) umgibt, um mindestens eine Gasprobe während des Mischzyklus zu erhalten, wobei dieser Schritt durch eine Geruchsdetektionsvorrichtung (120) des Systems (100) durchgeführt wird, die das Vorhandensein der geruchsbehafteten flüchtigen organischen Verbindungen (VOC), die in der in der Vorrichtung (120) aufgenommenen Umgebungsluft vorhanden sind, erkennt, indem sie ihre Konzentration durch Oberflächenplasmonenresonanz (SPR) misst;
- einen Schritt des Detektierens olfaktorischer Profile, die in der Umgebungsluft vorhanden sind und durch die Vorrichtung (120) erfasst werden;
- einen Schritt des Identifizierens (208) eines olfaktorischen Profils, um eine aus dem Mischmittel (112) austretende Mischung (M) und ihren Produktionszustand zu identifizieren, wobei dieser Schritt auf Grundlage mindestens einer Probe der durch die Vorrichtung (120) erfassten Umgebungsluft durchgeführt wird;
- einen Schritt des Analysierens (210) der durch die Vorrichtung (120) erfassten Proben auf Grundlage eines oder mehrerer durch die Vorrichtung (120) bereitgestellten SPR-Abdrücke (E_{A}, E_{B}) und der darin dargestellten extrahierten Daten;
- einen Schritt des Erstellens mindestens eines Modells olfaktorischer Profile auf Grundlage der physikalisch-chemischen Eigenschaften der identifizierten Kautschukmischung, wobei dieser Schritt einen Schritt des Eingebens (214), in ein neuronales Netzwerk, der physikalisch-chemischen Eigenschaften der identifizierten Kautschukmischung beinhaltet, wobei dieser Schritt durch das System (100) durchgeführt wird; und
- einen Schritt des Trainierens (216) des Modells olfaktorischer Profile auf Grundlage der olfaktorischen Profile der identifizierten Kautschukmischung;
sodass das sich ergebende Modell olfaktorischer Profile die Identifikation der physikalisch-chemischen Eigenschaften der Kautschukmischung ist, die gerade an der Produktionsanlage (110) produziert wird.

2. Verfahren (200) nach Anspruch 1, wobei der Schritt des Erstellens des Modells olfaktorischer Profile einen Schritt des Erzeugens einer Referenz der olfaktorischen Profile beinhaltet, die in der durch die Vorrichtung (120) zu erfassenden Umgebungsluft gesucht werden.

3. Verfahren (200) nach Anspruch 2, wobei der Schritt des Eingebens (214) des Schritts des Erstellens des Modells olfaktorischer Profile einen Schritt des Erzeugens einer Lerndatenbank (215) mit den physikalisch-chemischen Eigenschaften beinhaltet, die in das Modell olfaktorischer Profile eingegeben wird.

4. Verfahren (200) nach einem der Ansprüche 1 bis 3, ferner beinhaltend einen Schritt des Vergleichens (218), während dessen die Identifikation der physikalisch-chemischen Eigenschaften der aus dem Mischmittel (112) austretenden Mischung (M), die sich aus dem Modell olfaktorischer Profile ergibt, mit dem olfaktorischen Profil der identifizierten Mischung verglichen wird, sodass das System (100) die Produktionsanlage (110) anpassen kann, wenn die gewünschten Eigenschaften der Mischung (M) nicht erreicht werden.

5. Verfahren (200) nach Anspruch 4, ferner beinhaltend einen Schritt des Befehlens (220) einer Anpassung der Produktionsanlage (110), um das Erreichen der gewünschten Eigenschaften der aus dem Mischmittel (112) austretenden Mischung (M) vorzusehen.

6. Verfahren (200) nach Anspruch 5, wobei der Schritt des Befehlens (220) einer Anpassung der Produktionsanlage (110) einen Schritt des Bestimmens eines Klebrigkeitsniveaus der Mischung (M) auf Grundlage eines durch die Vorrichtung (120) bereitgestellten SPR-Abdrucks beinhaltet.

7. Verfahren (200) nach einem der Ansprüche 1 bis 6, wobei das System (100) während des Schritts des Trainierens (216) des Modells olfaktorischer Profile ein Lernverfahren einsetzt, das aus einem automatischen Lernverfahren und einem progressiven Lernverfahren ausgewählt wird.

8. Verfahren (200) nach einem der Ansprüche 1 bis 7, wobei:
- der Schritt des Detektierens olfaktorischer Profile einen Schritt des Beseitigens einer VOC-Verunreinigung der durch die Vorrichtung (120) aufgenommenen Umgebungsluft beinhaltet, wobei dieser Schritt durch einen Filter (140) des Systems (100) durchgeführt wird, bevor die Umgebungsluft in die Vorrichtung (120) eintritt;
- der Schritt des Identifizierens (208) eines olfaktorischen Profils einen Schritt des Verringerns der Feuchtigkeit und/oder der Temperatur in der durch die Vorrichtung (120) aufgenommenen Umgebungsluft beinhaltet, wobei dieser Schritt durch einen Kondensator (144) des Systems (100) durchgeführt wird; und
- der Schritt des Analysierens (210) der durch die Vorrichtung (120) erfassten Proben vor der durch die Vorrichtung (120) durchgeführten Analyse ferner einen Schritt des Analysierens olfaktorischer Profile in den Proben beinhaltet, wobei dieser Schritt durch einen Multiplexer (146) des Systems (100) durchgeführt wird.

9. Verfahren (200) nach einem der Ansprüche 1 bis 8, wobei der Schritt des Startens (202) eines Mischzyklus einen Schritt des Eingebens, in das Mischmittel (112), der für die Durchführung der Produktion der Mischung (M) erforderlichen Rohstoffe beinhaltet.

10. Verfahren (200) nach einem der Ansprüche 1 bis 8, wobei der Schritt des Startens (202) eines Mischzyklus einen Schritt des Eingebens, in das Mischmittel (112), eines oder mehrerer Masterbatches beinhaltet.

11. Verfahren (200) nach einem der Ansprüche 1 bis 10, ferner beinhaltend einen Schritt des Simulierens einer Anzahl von Mischungen, der es gestattet, mindestens eine Kautschukmischung (M) zu realisieren, die vorbestimmte physikalisch-chemische Eigenschaften aufweist.

12. System (100) zur Herstellung von Kautschukprodukten, das ein Verfahren (200) zur Steuerung der Herstellung von Kautschukprodukten durchführt, **dadurch gekennzeichnet, dass** das System (100) Folgendes beinhaltet:
- eine Produktionsanlage (110), die aufeinanderfolgende Schritte des Mischens umsetzt, wobei die Produktionsanlage mindestens ein Mischmittel (112) beinhaltet, aus dem eine oder mehrere Kautschukmischungen (M) austreten;
- eine Geruchsdetektionsvorrichtung (120), die Umgebungsluft, die die Produktionsanlage (110) umgibt, erfasst, um mindestens eine Gasprobe während des Mischzyklus zu erhalten; und
- ein Steuerungsteilsystem (130), das ein Modell olfaktorischer Profile auf Grundlage der physikalisch-chemischen Eigenschaften der Kautschukmischungen, die durch Oberflächenplasmonenresonanz (SPR) erkannt werden, einsetzt;
sodass die Vorrichtung (120) das Vorhandensein der geruchsbehafteten flüchtigen organischen Verbindungen (VOC), die in der erfassten Umgebungsluft vorhanden sind, erkennt, indem sie ihre Konzentration durch Oberflächenplasmonenresonanz (SPR) misst; und
sodass das Modell olfaktorischer Profile die physikalisch-chemischen Eigenschaften der VOC, die mit den aus dem Mischmittel (112) austretenden Mischungen (M) assoziiert sind, während der Produktionszyklen von Kautschukprodukten, die durch die Produktionsanlage (110) durchgeführt werden, lernt.

13. System (100) nach Anspruch 12, wobei das Teilsystem (130) einen oder mehrere Sensoren beinhaltet, die auslösen, wenn das sich ergebende Modell olfaktorischer Profile eine Diskrepanz zwischen den physikalisch-chemischen Eigenschaften der Mischung (M), die gerade an der Produktionsanlage (110) produziert wird, und den erwarteten physikalisch-chemischen Eigenschaften anzeigt.

14. System (100) nach Anspruch 13, wobei das Teilsystem (130) den Betrieb der Produktionsanlage (110) als Reaktion auf die ausgelösten Sensoren anpasst, um ein Klebrigkeitsniveau der Mischung (M) auf Grundlage eines durch die Vorrichtung (120) bereitgestellten SPR-Abdrucks zu erhalten.

15. System (100) nach einem der Ansprüche 12 bis 14, wobei das Mischmittel (112) aus einem oder mehreren Extrudern und/oder einem oder mehreren internen Mischern ausgewählt wird.

## Claims

1. A method (200) for controlling the manufacture of rubber products produced by a rubber-product manufacturing system (100) comprising a production facility (110) having at least one mixing means (112) that performs successive steps of mixing a rubber mixture, **characterized in that** the method comprises the following steps:
- a step (202) of initiating a mixing cycle that is performed at the production facility (110);
- a step (204) of capturing ambient air of the production facility (110) in order to obtain at least one gas sample during the mixing cycle, this step performed by an odor detection device (120) of the system (100) that recognizes the presence of the odorous volatile organic compounds (VOCs) present in the ambient air received in the device (120) by measuring its concentration using surface plasmon resonance (SPR);
- a step of detecting olfactory profiles present in the ambient air and captured by the device (120);
- a step (208) of identifying an olfactory profile in order to identify a mixture (M) exiting the mixing means (112) and the production status thereof, this step performed on the basis of at least one sample of the ambient air captured by the device (120);
- a step (210) of analyzing the samples captured by the device (120) against one or more SPR imprints (E_{A}, E_{B}) supplied by the device (120) and the extracted data represented therein;
- a step of constructing at least one olfactory-profiles model from the physicochemical properties of the identified rubber mixture, this step comprising a step (214) of introducing the physicochemical properties of the identified rubber mixture into a neural network, this step being performed by the system (100); and
- a step (216) of training the olfactory-profiles model using the olfactory profiles of the identified rubber mixture;
such that the output olfactory-profiles model will be the identification of the physicochemical properties of the rubber mixture in the process of being produced in the production facility (110).

2. The method (200) of Claim 1, wherein the step of constructing the olfactory-profiles model comprises a step of creating a reference of the olfactory profiles sought to be captured in the ambient air by the device (120).

3. The method (200) of Claim 2, wherein the introduction step (214) of the step of constructing the olfactory-profiles model comprises a step of creating a learning database (215) of the physicochemical properties that is introduced into the olfactory-profiles model.

4. The method (200) of any one of Claims 1 to 3, further comprising a comparison step (218) during which the identification of the physicochemical properties of the mixture (M) exiting the mixing means (112), output from the olfactory-profiles model, is compared against the olfactory profile of the identified mixture so that the system (100) can adjust the production facility (110) when the desired properties for the mixture (M) are not attained.

5. The method (200) of Claim 4, further comprising a step (220) of controlling the adjustment of the production facility (110) such that the mixture (M) exiting the mixing means (112) attains the desired properties.

6. The method (200) of Claim 5, wherein the step (220) of controlling the adjustment of the production facility (110) comprises a step of determining a level of tack of the mixture (M) from an SPR imprint supplied by the device (120).

7. The method (200) of any one of Claims 1 to 6, wherein, during the step (216) of training the olfactory-profiles model, the system (100) employs a learning method selected from a machine learning method and a progressive-learning method.

8. The method (200) of any one of Claims 1 to 7, wherein:
- the step of detecting olfactory profiles comprises a step of eliminating VOC pollution from the ambient air received by the device (120), this step performed by a filter (140) of the system (100) before the ambient air enters the device (120);
- the step (208) of identifying an olfactory profile comprises a step of reducing the moisture content and/or the temperature of the ambient air received by the device (120), this step performed by a condenser (144) of the system (100); and
- the step (210) of analyzing the samples captured by the device (120) further comprises a step of analyzing olfactory profiles in the samples prior to the analysis performed by the device (120), this step performed by a multiplexer (146) of the system (100).

9. The method (200) of any one of Claims 1 to 8, wherein the step (202) of initiating a mixing cycle comprises a step of introducing, into the mixing means (112), the raw materials needed for performing the production of the mixture (M).

10. The method (200) of any one of Claims 1 to 8, wherein the step (202) of initiating a mixing cycle comprises a step of introducing, into the mixing means (112), one or more masterbatches.

11. The method (200) of any one of Claims 1 to 10, further comprising a step of simulating a number of mixing operations yielding at least one rubber mixture (M) having predetermined physicochemical properties.

12. A system (100) for manufacturing rubber products that performs a method (200) for controlling the manufacture of rubber products, **characterized in that** the system (100) comprises:
- a production facility (110) that performs successive mixing steps, the production facility comprising at least one mixing means (112) from which one or more rubber mixture(s) (M) exit;
- an odor detection device (120) that captures ambient air of the production facility (110) in order to obtain at least one gas sample during the mixing cycle; and
- a control subsystem (130) that employs an olfactory-profiles model based on physicochemical characteristics of the rubber mixtures recognized by surface plasmon resonance (SPR);
such that the device (120) recognizes the presence of odorous volatile organic compounds (VOCs) present in the captured ambient air by measuring the concentration thereof using surface plasmon resonance (SPR); and
such that the olfactory-profiles model learns the physicochemical properties of the VOCs associated with the mixtures (M) exiting the mixing means (112) during the rubber-product production cycles performed by the production facility (110).

13. The system (100) of Claim 12, wherein the subsystem (130) comprises one or more sensors triggered when the output olfactory-profiles model indicates an offset between the physicochemical properties of the mixture (M) that is in the process of being produced in the production facility (110) and its expected physicochemical properties.

14. The system (100) of Claim 13, wherein the subsystem (130) adjusts the operation of the production facility (110) in response to the triggered sensors so as to obtain a level of tack of the mixture (M) from an SPR imprint supplied by the device (120).

15. The system (100) of any one of Claims 12 to 14, wherein the mixing means (112) is selected from one or more extruder(s) and/or one or more internal mixing mill(s).
